# EUROPÄISCHE PATENTANMELDUNG

(11) **EP 0 609 688 A2**
(43) Veröffentlichungstag der Anmeldung: **10.08.1994**
(21) Anmeldenummer: 94100612.4
(22) Anmeldetag: 18.01.1994
(51) Int. Cl.: G06F 15/42

(54) **Medizinisches Aggregat oder Gerät für Operationssäle, insbesondere Herz-Lungen-Maschine**

(30) Priorität: 21.01.1993 DE 4301524
(71) Anmelder: JOSTRA MEDIZINTECHNIK GmbH & Co. KG, D-72145 Hirrlingen (DE)
(72) Erfinder: Heinze, Werner, D-86923 Finning (DE)
(74) Vertreter: Möbus, Rudolf, Dipl.-Ing.

(57) **Zusammenfassung**

Bei dem medizinischen Aggregat oder Gerät für Operationssäle, insbesondere einer Herz-Lungen-Maschine, ist die elektronische Datenüberwachungs- und Datenauswerteeinrichtung in zwei elektrisch voneinander getrennte und in unterschiedlichen Räumen unterbringbare Einheiten aufgeteilt, nämlich in einen außerhalb des Operationssaales befindlichen Datenauswerteteil (15) und einen am Aggregat oder Gerät befindlichen Datenüberwachungs- und Datenspeicherteil (14). Der Datenspeicherteil besteht weitgehend aus mindestens einer Speicherkarte (20), für welche beide Einheiten (14) und (15) eine Schnittstelle oder Datenübertragungsstelle aufweisen. Dadurch läßt sich der Datenauswerteteil (15) für mehrere Aggregate oder Geräte verwenden und außerhalb des Operationssaales einfacher gestalten. Andererseits läßt sich das im Operationssaal befindliche Aggregat kompakter und raumsparender gestalten.

## Beschreibung

Die Erfindung betrifft ein medizinisches Aggregat oder Gerät für Operationssäle, insbesondere eine Herz-Lungen-Maschine, mit elektrisch betriebenen und/oder gesteuerten und/oder Daten liefernden Aggregat- oder Geräteteilen und mit einem elektronischen, Speicherstellen, mindestens einen Bildschirm und mindestens eine Zugriffstastatur aufweisenden, mit mindestens einem Mikroprozessor versehenen Datenüberwachungs- und Datenauswerteteil.

Medizinische Aggregate oder Geräte der eingangs genannten Art, die für schwierige Operationen eingesetzt werden und einen komplexen Aufbau haben, sind mit einem Datenauswerteteil versehen, der unter anderem auch den Ablauf einer Operation und die hierbei ermittelten und festgehaltenen Daten dokumentiert und nachträglich überprüfen läßt. Bisher ist dieser mit einem PC (Personalcomputer) versehene Datenauswerteteil beispielsweise bei Herz-Lungen-Maschinen mit im Operationssaal untergebracht und über ein Bündel von elektrischen Leitungen mit der übrigen elektronischen Steuereinrichtung und den gesteuerten Aggregat- oder Geräteteilen verbunden. Dies ist in mehrfacher Hinsicht nachteilig. Der Computer muß nach den strengen Sicherheitsbestimmungen in Operationssälen elektrisch modifiziert werden und ein besonderes Schutzgehäuse aufweisen, vergrößert den Platzbedarf des Aggregates und erfordert bauliche Maßnahmen zur Unterbringung der zahlreichen elektrischen Verbindungsleitungen, wenn sie ein Operationsteam bei seiner Arbeit nicht behindern sollen.

Der Erfindung liegt daher die Aufgabe zugrunde, ein medizinisches Aggregat oder Gerät der eingangs genannten Art so auszubilden, daß es einen kompakten, platzsparenden Aufbau ohne behindernde und freiliegende Verbindungsleitungen aufweist.

Die gestellte Aufgabe wird mit einem medizinischen Aggregat oder Gerät der eingangs genannten Art erfindungsgemäß dadurch gelöst, daß der elektronische Datenüberwachungs- und Datenauswerteteil in einen den Mikroprozessor mit Betriebsspeicher, einen Bildschirm und eine Zugriffstastatur aufweisenden Datenauswerteteil und einen Datenüberwachungs- und Datenspeicherteil, der vom Datenauswerteteil elektrisch getrennt ist, aufgeteilt ist und der Datenspeicherteil durch eine Speicherkarte gebildet ist.

Ein erfindungsgemäß ausgebildetes medizinisches Aggregat oder Gerät erbringt neben der Lösung der gestellten Aufgabe, also einen kompakten, raumsparenden Aufbau ohne behindernde Verbindungsleitungen zwischen getrennten Geräteteilen, auch einen rationelleren Einsatz und eine flexiblere Arbeitsweise mit dem Aggregat. Der Datenauswerteteil läßt sich außerhalb des Operationssaales in einem trockenen Arbeitsraum unterbringen, so daß gesonderte Schutzmaßnahmen entfallen und normale handelsübliche Computergeräte Verwendung finden können. Es genügt ein einziger Auswerteteil für mehrere medizinische Aggregate, deren Daten von der Speicherkarte des Datenüberwachungs- und Datenspeicherteiles der Aggregate über die leicht transportierbare und außerdem leicht datensichernd aufbewahrbare Speicherkarte dem Datenauswerteteil eingegeben werden können. Dies erleichtert auch das Auswechseln medizinischer Aggregate während eines Operationsvorganges und ein rasches Übergeben von Daten mittels der Speicherkarte auf ein Ersatzaggregat, mit welchem eine Operation weitergeführt wird. Die datenmäßige Vorbereitung einer Operation muß nicht an dem in einem Operationssaal angeordneten Aggregat oder Gerät vorgenommen werden, sondern die Speicherkarten können außerhalb mit den erforderlichen Patientendaten versehen werden.

Der Datenauswerteteil kann also vorteilhafterweise ein einfacher Personalcomputer mit einer Schnittstelle für Speicherkarten sein. Der vom Datenauswerteteil elektrisch getrennte Datenüberwachungs- und Datenspeicherteil kann seinerseits ein Anzeigefeld wahlweise für von den Aggregat- oder Geräteteilen gelieferte Daten oder von auf der Speicherkarte gehaltenen und vorgegebenen Festdaten aufweisen. Es ist somit eine sichere Kontrolle gegeben, daß auch die richtige, für eine bestimmte Operation vorbereitete Speicherkarte eingesetzt ist, und es kann eine laufende optische Überwachung der wichtigsten Daten erfolgen. Zweckmäßig kann der vom Datenauswerteteil elektrisch getrennte Datenüberwachungs- und Datenspeicherteil zusätzlich auch ein Tastenfeld für eine manuelle Betätigung oder Steuerung des Anzeigefeldes und/oder einzelner Aggregat- oder Geräteteile aufweisen, denen auf der Speicherkarte individuelle Speicherstellen zugeordnet sind. Somit erlaubt der Datenüberwachungs- und Datenspeicherteil auch eine nachträgliche und dokumentierte Änderung und Anpassung vorgegebener Werte oder der Ablauffolge von Operationsschritten. Die Speicherkarte weist also vorzugsweise gesonderte Speicherstellen für vorgebbare feste Daten und Speicherstellen für von den Aggregat- oder Geräteteilen gelieferte Perfusionsdaten auf, wobei die auf der Speicherkarte erfolgende laufende Datenaufzeichnung von einem Zeitgeber beeinflußt und abhängig ist.

Nachfolgend wird ein Ausführungsbeispiel eines erfindungsgemäß ausgebildeten medizinischen Aggregates, nämlich eine Herz-Lungen-Maschine, anhand der beiliegenden Zeichnung mit ihren erfindungswesentlichen Teilen näher erläutert.

Im einzelnen zeigen:
- Fig. 1: eine schematische Darstellung der elektrisch getrennten Anordnung von Einheiten der elektronischen Datenüberwachungs- und Datenauswerteeinrichtung einer Herz-Lungen-Maschine;
- Fig. 2: eine perspektivische Übersichtsdarstellung der im Operationssaal verbleibenden Einheit der Herz-Lungen-Maschine nach Fig. 1.

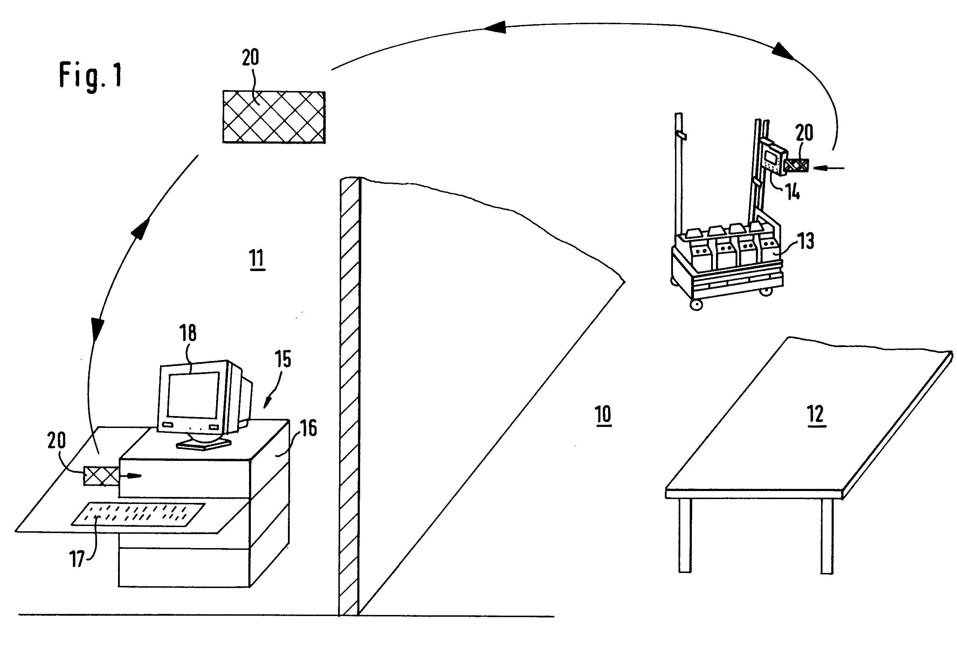

Fig. 1 zeigt einen Operationssaal 10 und einen anderen Raum 11 einer Klinik. In dem Operationssaal sind schematisch ein Operationstisch 12 und eine Herz-Lungen-Maschine 13 dargestellt. Die elektronische Datenüberwachungs- und Datenauswerteeinrichtung der Herz-Lungen-Maschine 13 ist in zwei elektrisch voneinander getrennte Einheiten aufgeteilt, nämlich in einen an der Herz-Lungen-Maschine 13 im Operationssaal 10 befindlichen Datenüberwachungs- und Datenspeicherteil 14 und einen in dem anderen Raum 11 der Klinik befindlichen Datenauswerteteil 15. Der Datenauswerteteil 15 weist einen handelsüblich ausgestatteten Personalcomputer 16 mit einer Bedienungstastatur 17 und einem Bildschirm 18 auf. Koppelungsglied zwischen den beiden Einheiten 14 und 15 ist ein mobiler Datenspeicherteil in Form mindestens einer ohne äußere Kontakte ausgebildeten Speicherkarte 20 (Memory Card). Diese Speicherkarte 20 befindet sich während des Betriebs der Herz-Lungen-Maschine 13 im Operationssaal in der Einheit 14. Zur Datenauswertung und auch zur Bestückung der Speicherkarte 20 mit einen Patienten und Teile des medizinischen Aggregates oder Gerätes betreffenden Festdaten wird die Speicherkarte 20 in die Einheit 15 eingegeben.

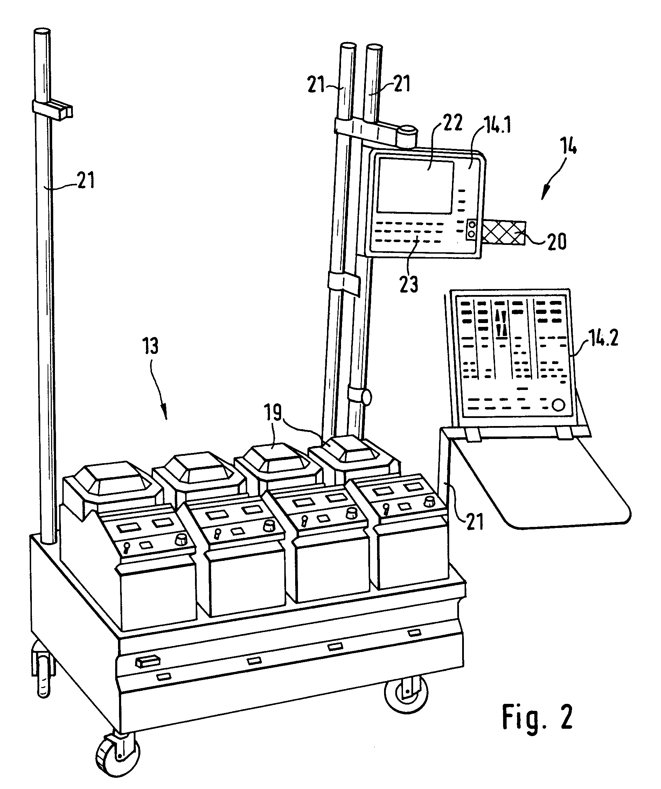

Fig. 2 zeigt die Herz-Lungen-Maschine 13 mit der elektronischen Datenüberwachungs- und Datenspeichereinheit 14 in Einzeldarstellung. Die Herz-Lungen-Maschine 13 ist mit vier Pumpen 19 bestückt. Die elektrischen Steuerleitungen zwischen diesen und anderen Aggregatteilen der Herz-Lungen-Maschine 13 mit der elektronischen Einheit 14 verlaufen nach außen abgeschirmt über Haltemasten 21 der verfahrbaren Maschine 13. Die elektronische Einheit 14 ist beim dargestellten Ausführungsbeispiel in zwei Teile 14.1 und 14.2 gegliedert. Der Teil 14.1 weist einen Flachbildschirm als Anzeigefeld 22, ein Tastenfeld 23 und eine Aufnahmestelle für die Speicherkarte 20 auf. Das Tastenfeld 23 weist sogenannte Ereignistasten auf, über welche gesonderte, auf der Speicherkarte 20 oder in internen Speicherstellen befindliche oder während eines Operationsablaufes auftretende Daten zur Darstellung im Anzeigefeld 22 oder zur Datenänderung aufrufbar sind. Die Speicherkarte selbst ist in verschiedene Speicherblöcke unterteilt, die entweder festliegende und bereits vor einer Operation in der Einrichtungseinheit 15 eingebbare Festdaten oder während der Operation auftretende Perfusionsdaten speichern oder mit den Ereignistasten des Tastenfeldes 23 in Verbindung stehen. Der Teil 14.2 der Datenüberwachungs- und Datenspeichereinheit 14 weist überwiegend Bedienungstasten und Anzeigevorrichtungen für einzelne Aggregatteile auf. Eine Zeitgeberstufe der elektronischen Einrichtung kann entweder im Teil 14.1 oder im Teil 14.2 untergebracht sein.

Alle vier dargestellten Pumpen 19 der Herz-Lungen-Maschine, wie auch andere, nicht dargestellte elektrisch betriebene oder überwachte Aggregatteile, sind unter Vermeidung von freiliegenden Verbindungskabeln über Steckkupplungen auswechselbar angeordnet, von denen der eine Kupplungsteil unmittelbar an dem Aggregatteil und der andere Kupplungsteil unmittelbar an einem das Aggregatteil haltenden oder tragenden Gehäuseteil der Herz-Lungen-Maschine 13 angeordnet ist.

## Patentansprüche

1. Medizinisches Aggregat oder Gerät für Operationssäle, insbesondere Herz-Lungen-Maschine, mit elektrisch betriebenen und/oder gesteuerten und/oder Daten liefernden Aggregat- oder Geräteteilen und mit einer elektronischen, Speicherstellen, mindestens einen Bildschirm und mindestens eine Zugriffstastatur aufweisenden, mit mindestens einem Mikroprozessor versehenen Datenüberwachungs- und Datenauswerteeinrichtung, dadurch gekennzeichnet, daß die elektronische Datenüberwachungs- und Datenauswerteeinrichtung in einen den Mikroprozessor mit Betriebsspeicher, einen Bildschirm (18) und eine Zugriffstastatur (17) aufweisenden Datenauswerteteil (15) und einen Datenüberwachungs- und Datenspeicherteil (14), der vom Datenauswerteteil (15) elektrisch getrennt ist, aufgeteilt und der Datenspeicherteil (14) durch mindestens eine Speicherkarte (20) gebildet ist.

2. Medizinisches Aggregat oder Gerät nach Anspruch 1, dadurch gekennzeichnet, daß der Datenauswerteteil (15) einen Personalcomputer (16) mit einer Schnitt stelle für kontaktlose Speicherkarten (20) aufweist.

3. Medizinisches Aggregat oder Gerät nach Anspruch 1 oder 2, dadurch gekennzeichnet, daß der vom Datenauswerteteil (15) elektrisch getrennte Datenüberwachungs- und Datenspeicherteil (14) ein Anzeigefeld (22) wahlweise für von den Aggregat- oder Geräteteilen (19) gelieferte Daten oder von auf der Speicherkarte (20) gehaltenen und vorgegebenen Festdaten aufweist.

4. Medizinisches Aggregat oder Gerät nach Anspruch 3, dadurch gekennzeichnet, daß der vom Datenauswerteteil (15) elektrisch getrennte Datenüberwachungs- und Datenspeicherteil (14) zusätzlich mindestens ein Tastenfeld (23) für eine manuelle Betätigung oder Steuerung des Anzeigefeldes (22) und/oder einzelner Aggregat- oder Geräteteile (19), denen auf der Speicherkarte individuelle Speicherstellen zugeordnet sind, aufweist.

5. Medizinisches Aggregat oder Gerät nach einem der Ansprüche 1 bis 4, dadurch gekennzeichnet, daß die Speicherkarte (20) Speicherstellen für vorgebbare feste Daten und Speicherstellen für von den Aggregat- oder Geräteteilen gelieferte Perfusionsdaten aufweist.

6. Medizinisches Aggregat oder Gerät nach einem der Ansprüche 1 bis 5, dadurch gekennzeichnet, daß in dem vom Datenauswerteteil (15) elektrisch getrennten Datenüberwachungs- und Datenspeicherteil (14) die Datenspeicherung auf der Speicherkarte (20) von einem Zeitgeber beeinflußt und abhängig ist.
